Europäisches Patentamt

European Patent Office

Office européen des brevets

(19)

(11) Veröffentlichungsnummer: **0 197 403**
**B1**

(12) EUROPÄISCHE PATENTSCHRIFT

(45) Veröffentlichungstag der Patentschrift: **13.06.90**

(21) Anmeldenummer: **86103942.8**

(22) Anmeldetag: **22.03.86**

(51) Int. Cl.⁵: **C 07 C 29/04,** B 01 J 31/10,
B 01 J 35/10

(54) **Verfahren zur kontinuierlichen Herstellung von niederen Alkoholen.**

(30) Priorität: **06.04.85 DE 3512518**

(43) Veröffentlichungstag der Anmeldung:
**15.10.86 Patentblatt 86/42**

(45) Bekanntmachung des Hinweises auf die
Patenterteilung:
**13.06.90 Patentblatt 90/24**

(84) Benannte Vertragsstaaten:
**AT BE DE FR GB IT NL SE**

(56) Entgegenhaltungen:
EP-A-0 084 133
DE-C-3 040 997
FR-A-1 573 164
FR-A-2 398 711
FR-A-2 470 105
GB-A-2 081 254
US-A-3 328 471

(73) Patentinhaber: **RWE-DEA Aktiengesellschaft für
Mineraloel und Chemie
Überseering 40
D-2000 Hamburg 60 (DE)**

(72) Erfinder: **Henn, Friedrich
Erlenstrasse 10
D-4100 Duisburg 17 (DE)**
Erfinder: **Neier, Wilhelm
An den Landwehr 40⁻
D-4134 Rheinberg 3 (DE)**
Erfinder: **Strehlke, Günter
Schlotweg 4
D-4100 Duisburg 74 (DE)**
Erfinder: **Webers, Werner
Rektor-Horn-Strasse 42
D-4134 Rheinberg 3 (DE)**

(74) Vertreter: **Schupfner, Gerhard D.
Müller, Schupfner & Gauger Karlstrasse 5
Postfach 14 27
D-2110 Buchholz/Nordheide (DE)**

**Beschreibung**

Die Erfindung bezieht sich auf ein Verfahren zur kontinuierlichen Herstellung von niederen Alkoholen mit 3—5 Kohlenstoffatomen durch katalytische Hydratation von niederen Olefinen mit 3—5 Kohlenstoffatomen in Gegenwart eines starksauren Kationenaustauschers als Katalysator, der als Festbett angeordnet ist, bei einer Temperatur von etwa 120—180°C und einem Druck von etwa 40—200 bar, Abführung des Produktstroms und Abtrennung des gebildeten Alkohols aus dem Produkstrom sowie Rückführung des Prozeßwassers.

Solche Verfahren sind unter anderem in den deutschen Patentschriften 22 33 967 und 24 29 770 beschrieben und dienen insbesondere zur Herstellung von Isopropylalkohol (IPA), sec.-Butylalkohol (SBA) und tert.-Butylalkohol (TBA).

Führt man die Hydratisierung zum Beispiel von n-Butenen nach dem in der DE—PS 24 29 770 beschriebenen Verfahrensweise durch und leitet das Prozeßwasser zurück, etwa gemäß DE—PS 30 40 997, so steigt nach einer Laufzeit von 200—400 Stunden der Differenzdruck zwischen Reaktorsumpf und Reaktorkopf ständig an. Im weiteren Verlauf treten starke Druckstöße auf, die zum Abstellen des Reaktors und Spülen mit Wasser zwingen.

Ständiges Auskreisen von Prozeßwasser jedoch und Verwendung von frischem Wasser zur Beherrschung des Druckproblems verbietet sich aus wirtschaftlichen Überlegungen, und das Aufbereiten des zurückzuführenden Prozeßwassers an Ionenaustauschern bringt in Bezug auf die Senkung des Differenzdruckes keine Verbesserung.

Auch beim Rieselverfahren gemäß de DE—PS 22 33 967 bestehen Verteilungsprobleme. Hinweise darauf, daß eine bessere Verteilung der Reaktanden angestrebt werden sollte, ergeben sich insbesondere beim Anfahren des Betriebsreaktors nach einer Neubefüllung mit frischem Kontakt. Heirbei wird eine Hot Spot-Bildung beobachtet, die sich dann als besondere Geruchsnote im destillierten Isopropylalkohol bemerkbar macht. Es wird angenommen, daß sich in der Anfahrphase an besonders reaktiven Stellen des Katalysators, verbunden mit einer partiell schlechten Verteilung des Wassers, Nebenreaktionen abspielen, die zu einer regelrechten Verbrennung unter Verklumpung von Katalysatorbezirken führen.

Der vorliegenden Erfindung liegt daher die Aufgabe zugrunde, das Verfahren der Direkthydratation von niederen Olefinen so durchzuführen, daß eine Verbesserung der Verteilung der Reaktanden am Katalysator erziélt und der mit zunehmender Laufzeit stetig ansteigende Differenzdruck auf niederem Druckniveau stabilisiert wird.

Die Aufgabe wird erfindungsgemäß dadurch gelöst, daß man dem Prozeßwasser ein unter den Verfahrensbedingungen stabiles Kationentensid in einer Konzentration von mindestens etwa 2 ppm bis etwa 100 ppm zufügt und diese Tensidkonzentration während des Verfahrens aufrechterhält.

Bevorzugt werden Tensidkonzentrationen von 2 bis 40 eingesetzt. Konzentrationen über 100 ppm sind möglich, jedoch aus wirtschaftlichen Gründen in der Regel nicht zweckmäßig.

Gemäß einer bevorzugten Ausführungsforme des erfindungsgemäßen Verfahrens wird der als Festbett angeordnete Katalysator von mindestens einer Reaktionskomponente von unten nach oben durchströmt, die Gasphase am Kopf des Reaktors abgeführt und der gebildete Alkohol aus der Gasphase durch partielle Entspannung abgetrennt.

Es wurde überraschenderweise gefunden, daß es nicht zum Ansteigen des Differenzdrucks kommt, wenn man dem Prozeßwasser kontinuierlich eine geringe Menge eines kationischen Tensids, insbesondere von Typ der quartären Ammoniumverbindungen zufügt.

Nichtionische oder anionische Tenside zeigen diesen Effekt nicht. Anionische Tenside zeigen sogar einen gegenteiligen Effekt, das heißt beim Zudosieren von Anionentensiden zum Prozeßwasser kommt es zu einem weiteren Ansteigen des Differenzdruckes.

Zur Durchführung des erfindungsgemäßen Verfahrens sind grundsätzlich alle Kationentenside geeignet, die unter den Reaktionsbedingungen des Verfahrens der Direkthydratation stabil und zwar insbesondere temperaturstabil sind, so z.B. Verbindung der allgemeinen Formel $RR_1R_2R_3N^\oplus X^\ominus$ worin R, $R_1$, $R_2$ und $R_3$ Alkyl- oder Alkanoyl-Reste mit 1—18 C-Atomen und $X^\ominus$ ein Anion, insbesondere Cl bedeuten, insbesondere die Verbindungen Dimethyldistearylammoniumchlorid, Trimethylpalmitylammoniumchlorid, Cetyltrimethylammoniumchlorid, Lauryldimethylbenzylammoniumchlorid, Oxyethylalkylammoniumphosphat.

Alkanolammoniumsalze, Pyridiniumsalze, Imidazolinsalze, Oxazoliniumsalze, Thiazoliniumsalze, Salze von Aminoxiden, Sulfoniumsalze, Chinoliniumsalze, Isochinoliniumsalze, Tropyliumsalze können ebenfalls wirksam sein. Ihre Temperaturstabilität mag allerdings geringer sein, als die der an erster Stelle angeführten Ammoniumverbindungen.

Dimethyldistearylammoniumchlorid erweist sich als besonders wirksam und besitzt den Vorteil einer besonders guten Temperaturstabilität.

Es war besonders überraschend, daß der positive Effekt schon bei sehr geringen Tensidkonzentrationen eintritt. Bereits eine Menge von 4—10 ppm, die kontinuierlich dem Prozeßwasser zugesetzt wird, senkte nachhaltig den Differenzdruck und hielt ihn auf niederem Niveau konstant.

Grundsätzlich desaktivieren die Kationentenside die Kationenaustauscher. Hier wurde allerdings festgestellt, daß die wirksame Dosierrate des eingesetzten Kationentensids so gering ist, daß keine messbare Deaktivierung des als Katalysator verwendeten starksauren Kationenaustauschers beobachtet wird. Im Gegenteil, es kommt zu einer Reihe von positiven Effekten, wie bessere Verteilung der Reaktanden auf dem Katalysator und Stabilisierung des Differenzdruckes auf niederem Niveau, die die Verfahren zur Herstellung niederer Alkohole durch Verbesserung von Selektivität und Produktqualität noch wirtschaftlicher gestalten. Durch die Zugabe des Kationentensids findet eine bessere Phasenverteilung am Kontaktkorn statt. Deutliches Indiz ist bei der sec-Butylalkohol-Herstellung die durch das erfindungsgemäße Verfahren erzielte Senkung der gebildeten Menge an Di-sec-butylether (SBE) von etwa 4 Gewichtsteilen auf 1 Gewichtsteil, bezogen auf sec-Butylalkohol. Die Trennung der Gas- und Flüssigphase am Kopf des Reaktors wird verbessert. Die besonders bei hoher Gasquerschnittsbelastung zu beobachtende Schaumbildung wird völlig unterdrückt und der erhaltene Rohalkohol ist frei von durch Schaum mitgerissenen Säurespuren aus dem Prozeßwasser.

Die Durchführung des erfindungsgemäßen Verfahrens erfolgt in wäßriger Phase. Es werden keine organischen Lösungsmittel zugesetztwenn man davon absieht, daß die Tenside in wäßriger und/oder alkoholischer Lösungs eingebracht werden können.

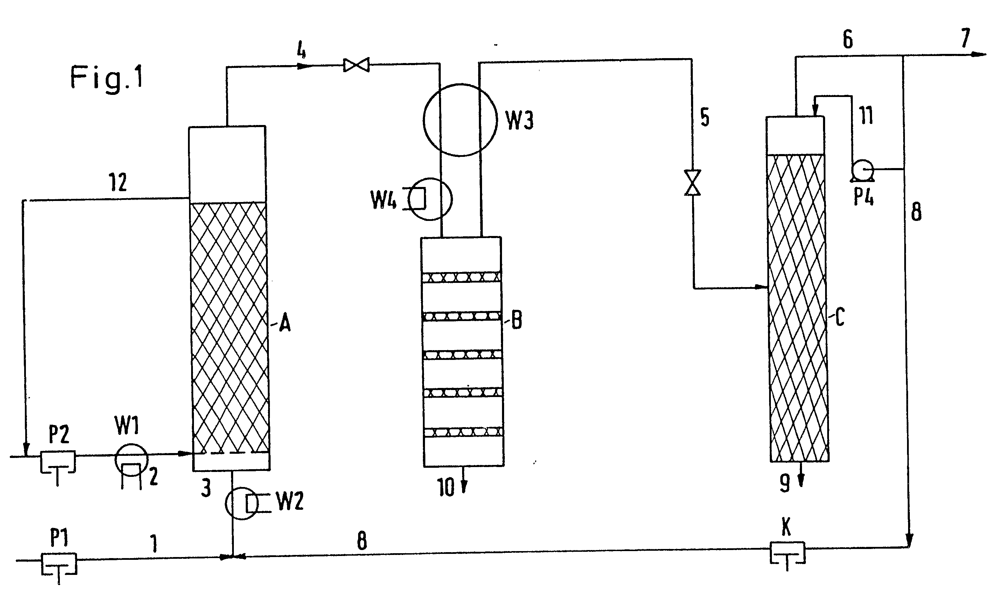

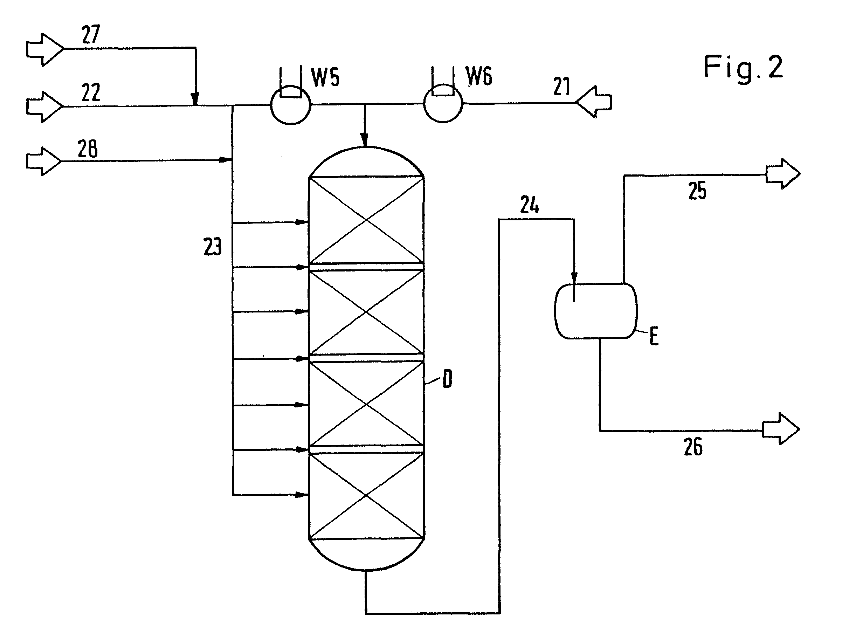

Im folgenden werden Ausführungsbeispiele der Erfindung anhand der beigefügten Figuren 1 und 2 beschrieben.

Es zeigen

Fig. 1 ein Fließschema zur Durchführung eines Sumpfphasen-Verfahrens gemäß der DE—PS 30 40 997 und

Fig. 2 ein Fließschema zur Durchführung eines Rieselverfahrens gemäß der DE—PS 22 33 967.

Die folgenden Beispiele erläutern das erfindungsgemäße Verfahren.


Vergleichsbeispiel 1

Der in Fig. 1 gezeigte Reaktor A hatte einen Durchmesser von 500 mm une eine Länge von 10,0 m. Er war mit 1.700 l eines starksauren Ionenaustauschers gefüllt. Der Differenzdruck über die Reaktorlänge wurde mitels Differenzdruckmessung verfolgt.

Dem Reaktor wurden stündlich über Leitung 1 300,3 kg eines mit 1,5% Wasser vorgesattigten 85% n-Buten enthaltenden n-Buten/Butan-Gemisches (4.534 Mole n-Butene) und über Leitung 2 204 kg (11.333 Mole $H_2O$) entmineralisiertes Wasser eingespeist. Das Wasser wurde auf eine Temperatur von 155—160°C im Wärmetauscher W1 vorgeheizt. Das über Leitung 1 mit Hilfe der Dosierpumpe P1 zudosierte Einsatzgas wurde mit dem über Leitung 8 zurückgeführten Kreislaufgas gemischt und im Verdampfer W2 verdampft, auf eine Temperatur von 160°C gebracht und am Sumpf über Leitung 3 in den Reaktor eingeleitet. Im Reaktor wurde ein Reaktionsdruck von 60 bar aufrechterhalten. Am Kopf des Reaktors wurde der gebildete sec.-Butylalkohol dampfförmig zusammen mit dem überschüssigen $C_4$-Gas über Leitung 4 abgezogen, entspannt und verflüssigt und über Wärmetauscher W3 und W4 durch den Abscheider B geleitet. Das abgeschiedene Wasser wird über Leitung 10 abgezogen und als Teil des Prozeßwassers über Leitung 2 erneut eingesetzt. Das flüssige n-Butan/n-Buten-Gemisch wird durch Gegentausch im Wärmetauscher W3 wieder verdampft und über Leitung 5 in der Druckkolonne C in Alkohol und Reaktionsgas getrennt. Der Alkohol wird über Leitung 9 abgezogen. Das überschüssige n-Butan/n-Buten-Gemisch wurde zum überwiegenden Teil (2.700 kg/h) mit Hilfe des Kompressors K über Leitung 8 dem Reaktor erneut zugeführt. Über Leitung 7 wurde ein Teilstrom als Restgas ausgeschleust. Mit der Pumpe P4 wird ein kleiner Rückfluß 11 zur Kolonne gegeben.

Folgende Ergebnisse wurden erhalten:

| Betriebszeit Tage | Differenzdruck bar | Menge SBA kg/h | Menge SBE kg/h |
|---|---|---|---|
| 1 | 2,4 | 202 | 2,0 |
| 3 | 3,5 | 190 | 2,7 |
| 5 | 3,7 | 188 | 3,1 |
| 7 | 3,7 | 189 | 3,0 |
| 9 | 3,6 | 190 | 3,2 |
| 10 | 3,8 | 188 | 3,0 |

SBA sec.-Butylalkohol
SBE Di-sec.-butylether


Am Abscheider B wurden über Strom 10 etwa 55 kg/h einer wäßrigen Phase erhalten.

Vergleichsbeispiel 2

Das im Vergleichsbeispiel 1, beschriebene Verfahren wurde mit der Änderung wiederholt, daß am Kopf abgezogenes Prozeßwasser am Reaktorsumpf erneut eingespeist wurde. Die Hauptmenge das Prozeßwassers, etwa 96%, wurde über Leitung 12 zurückgeführt. Über Leitung 10 wurden etwa 4% des Prozeßwassers zurückgeführt. Unter sonst gleichen Bedingungen wurden folgende Ergebnisse erhalten:

| Betriebszeit Tage | Differenzdruck bar | Menge SBA kg/h | Menge SBE kg/h |
|---|---|---|---|
| 1 | 2,7 | 203 | 2,1 |
| 3 | 3,6 | 189 | 2,9 |
| 5 | 6,7 | 181 | 5,8 |
| 7 | 8,9 | 174 | 7,8 |
| 9 | 9,6 | 171 | 8,6 |
| 10 | 10,1 | 171 | 8,8 |

Nach einer Druckentspannung auf 1 bar und Abstellen der Gaseinspeisung wurde de Katalysator 24 Std. mit reinem Wasser gewaschen.

Nach Versuchsfortsetzung wurden folgende Ergebnisse erzielt:

| Betriebszeit Tage | Differenzdruck bar | Menge SBA kg/h | Menge SBE kg/h |
|---|---|---|---|
| 11 | 2,9 | 202 | 2,3 |
| 13 | 3,9 | 188 | 3,1 |
| 15 | 7,2 | 179 | 6,0 |
| 17 | 9,3 | 172 | 8,1 |
| 19 | 10,1 | 170 | 8,7 |
| 20 | 10,8 | 169 | 9,0 |

Über Strom 10 wurden etwa 80 kg/h einer wäßrigen Phase abgezogen.

Beispiel 1

Das Vergleichsbeispiel 2 wurde mit der Maßgabe wiederholt, daß dem Prozeßwasserstrom (204 kg/h) Vor der Pumpe P2 stündlich 8 g einer 20 %igen Lösung von Dimethyldistearylammoniumchlorid in SBA zugesetzt wurden. Das Prozeßwasser wies damit eine Tensidkonzentration von 8 ppm auf. 70—80% der Tensidmenge verblieben auf dem Katalysator.

Folgende Engebnisse wurden erhalten:

4

# EP 0 197 403 B1

| Betriebszeit Tage | Differenzdruck bar | Menge SBA kg/h | Menge SBE kg/h |
|---|---|---|---|
| 1 | 0,6 | 208 | 1,9 |
| 3 | 0,8 | 207 | 1,8 |
| 5 | 0,7 | 207 | 1,7 |
| 7 | 0,7 | 208 | 1,8 |
| 9 | 0,8 | 207 | 1,8 |
| 11 | 0,8 | 208 | 1,9 |
| 15 | 0,7 | 208 | 1,8 |
| 20 | 0,8 | 208 | 1,8 |
| 30 | 0,8 | 207 | 1,8 |

## Beispiel 2

Beispiel 1 wurde mit der Maßgabe wiederholt, daß die Dosierung der 20 %igen Lösung des kationischen Tensides auf 80 g/h erhoht wurde. Über 30 Tage wurden die gleichen Ergebnisse erhalten wie im Beispiel 1.

## Vergleichsbeispiel 3

Der in Fig. 2 gezeigte Reaktor D hat einen Durchmesser von 500 mm und eine Länge von 10 m. Er war mit 1.700 l eines starksauren Kationenaustauschers gefüllt. Zur Abführung der Reaktionswärme wurde gemäß der DE—PS 21 47 739 ein Teil des Reaktionswassers an getrennten Stellen in das Katalysatorbett eingeleitet. Der Differenzdruck über die Reaktorlänge wurde mittels Differenzdruckmessung verfolgt.

Dem Reaktor wurden stündlich uber Leitung 21 331 kg eines 92% Propen enthaltenden Propen/Propan-Gemisches (7.250 Mole Propen) und über Leitung 22 2.475 kg (137,5 Kmol $H_2O$) entmineralisiertes Wasser eingespeist.

Ein Teil des Wassers wurde im Wärmetauscher W5 auf 140—145°C vorgeheizt. Ein anderer Teil (ca 25%) wurde ohne Vorheizung zur Abführung der Reaktionswärme an verschiedenen Stellen dem Reaktor zugeführt. Das über Leitung 21 dosierte Einsatzgas wurde im Verdampfer W6 verdampft und gemeinsam mit dem Reaktionswasser am Kopf des Reaktors eingespeist. Im Reaktor wurde ein Reaktionsdruck von 100 bar eingestellt. Über Leitung 24 wurde der wäßrige Isopropylalkohol zusammen mit dem überschüssigen Reaktionsgas in den Abscheider E gegeben. Nach Abtrennung der Gasphase (Ableitung über Leitung 25) wurden über Leitung 26 stündlich 2.690 kg eines wäßrigen Isopropylalkohols gewonnen, der 295 kg Isopropylalkohol und 25,6 kg Di-isopropylether enthielt. Die Katalysatorleistung betrug 2,89 Mol IPA/l Kat × h, die Selektivität lag bei 92%. Über die Reaktorlänge wurden ein Differenzdruck zwischen 3,5 und 4,0 bar gemessen.

## Beispiel 3

Das Vergleichsbeispiel 3 wurde unter sonst gleichen Bedingungen mit der Maßgabe wiederholt, daß über die Leitungen 27 und 28 der Leitung 22 stündlich 62 g und der Leitung 23 stündlich weitere 62 g einer 20% igen Lösung Dimethyldistearylammoniumchlorid in Isopropylalkohol zudosiert wurde.

Durch diese Maßnahme sank der Differenzdruck über die Reaktorlänge auf 1,6—2,0 bar ab. Die erzeugte Menge Isopropylalkohol stieg auf 327 kg/h an und die gebildete Ethermenge nahm auf 15,0 kg/h ab.

Die Katalysatorleistung betrug jetzt 3,20 Mol IPA/l Kat × h, die Selektivität lag bei 95,6%.

**Patentansprüche**

1. Verfahren zur kontinuierlichen Herstellung von niederen Alkoholen mit 3—5 Kohlenstoffatomen durch katalytische Hydratation von niederen Olefinen mit 3—5 Kohlenstoffatomen in Gegenwart eines starksauren Kationenaustauschers als Katalysator, der als Festbett angeordnet ist, bei einer Temperatur von etwa 120—180°C und einem Druck von etwa 40—200 bar, Abführung des Produktstroms und Abtrennung des gebildeten Alkohols aus dem Produktstrom sowie Rückführung des Prozeßwassers, dadurch gekennzeichnet, daß man dem Prozeßwasser ein unter den Verfahrensbedingungen stabiles Kationentensid in einer Konzentration von mindestens etwa 2 ppm bis etwa 100 ppm zufügt und diese Tensidkonzentration während des Verfahrens aufrechterhält.

5

## EP 0 197 403 B1

2. Verfahren nach Anspruch 1, dadurch gekennzeichnet, daß man als Tensid quartäre Ammoniumverbindungen einsetzt.

3. Verfahren nach Anspruch 2, dadurch gekennzeichnet, daß man als Tensid Dimethyldistearylammoniumchlorid eingesetzt.

4. Verfahren nach einem der Ansprüche 1 bis 3, dadurch gekennzeichnet, daß man eine Tensidkonzentration von 2—40 ppm, und vorzugsweise 4 bis 10 aufrechterhält.

5. Verfahren nach einem der vorhergehenden Ansprüche, dadurch gekennzeichnet, daß man den als Festbett angeordneten Katalysator zumindest von einer Reaktionskomponente von unten nach oben durchströmen läßt, die Gasphase am Kopf des Reaktors abführt und den gebildeten Alkohol aus der Gasphase durch partielle Entspannung abtrennt.

### Revendications

1. Procédé de préparation continue de bas alcools contenant de 3 à 5 atomes de carbone par l'hydratation catalytique de basses oléfines contenant de 3 à 5 atomes de carbone, en présence d'un échangeur de cations fortement acid disposé sous forme d'un lit fixe, à une température d'environ 120—180°C et une pression d'environ 40—200 bars, avec soutirage du courant du produit, séparation de l'alcool de ce courant et recyclage de l'eau de procédé, caractérisé par le fait que l'on ajoute à l'eau de procédé au moins 2 à 100 ppm d'un agent tensioactif cationique qui reste stable dans le conditions de service et que l'on maintient ladite concentration durant le procédé.

2. Procédé selon la revendication 1, caractérisé par le fait que l'on utilise comme agent tensioactif un composé d'ammonium quaternaire.

3. Procédé selon la revendication 2, caractérisé par le fait que l'on utilise comme agent tensioactif le chlorure de diméthyldistéarylammonium.

4. Procédé selon l'une des revendications 1 à 3, caractérisé par le fait que l'on maintient la concentration de l'agent tensioactif à 2—40 ppm, de préférence, à 4—10 ppm.

5. Procédé selon l'une des revendications précédentes, caractérisé par le fait que le catalyseur est disposé sous forme d'un lit fixe, qu'au moins un constituant de la charge de réactifs passe à travers le réacteur de bas en haut, que la phase gazeuse est déchargée au sommet du réacteur et que l'alcool produit est séparé de la phase gazeuse par détente partielle.

### Claims

1. A process for the continuous production of lower alcohols having 3 to 5 carbon atoms by the catalytic hydration of aliphatic olefins having 3 to 5 carbon atoms in the presence of a strongly acidic cation exchange catalyst in a fixed bed reactor at a temperature from about 120—180°C and a pressure from about 40—200 bar, with removal of the product stream, separation of the alcohol from the product stream and recycling of the process water, characterised by adding a cationic surfactant which is stable under the process conditions in a concentration ranging from about 2 to 100 ppm to the process water and maintaining this concentration throughout the process.

2. A process according to claim 1 in which said surfactant is a quaternary ammonium compound.

3. A process according to claim 2 in which said surfactant is dimethyl distearyl ammonium chloride.

4. A process according to any one of claims 1 to 3 in which the concentration of the surfactant is maintained in a range from about 2 to 40 ppm, preferably from 4 to 10 ppm.

5. A process according to any one of the preceding claims characterised by passing at least one reaction component from the bottom to the top of the fixed bed catalyst, removing the gaseous phase at the head of the reactor and separating the alcohol from the gaseous phase by partial pressure release.

6

Fig.1

Fig.2

EP 0 197 403 B1